Europäisches Patentamt

European Patent Office

Office européen des brevets

(11) Veröffentlichungsnummer: **0 256 444 B1**

## EUROPÄISCHE PATENTSCHRIFT

(45) Veröffentlichungstag der Patentschrift: **09.09.92**

(21) Anmeldenummer: **87111447.6**

(22) Anmeldetag: **07.08.87**

(51) Int. Cl.⁵: **G01N 1/00**, G01N 33/18, G01N 33/00

(54) **Einrichtung zur Überwachung leichtflüchtiger chlorierter und fluorierter Kohlenwasserstoffe.**

(30) Priorität: **12.08.86 DE 8621636 U**

(43) Veröffentlichungstag der Anmeldung:
**24.02.88 Patentblatt 88/08**

(45) Bekanntmachung des Hinweises auf die
Patenterteilung:
**09.09.92 Patentblatt 92/37**

(84) Benannte Vertragsstaaten:
**AT BE CH DE FR GB IT LI LU NL SE**

(56) Entgegenhaltungen:
**DE-B- 1 773 510**
**DE-B- 2 260 088**
**DE-B- 2 713 621**
**US-A- 3 942 792**
**US-A- 4 154 086**

**SIEMENS POWER ENGINEERING, Band 5, Nr. 6, November-Dezember 1983, Seiten 286,287, Passau, D; D. SAJONZ: "Continuous Monitoring of Water for Volatile Hydrocarbons"**

(73) Patentinhaber: **AUERGESELLSCHAFT GMBH**
**Thiemannstrasse 1**
**W-1000 Berlin 44(DE)**

(72) Erfinder: **Melzer, Werner, Dr.**
**Drosselweg 2**
**W-6237 Liederbach(DE)**

# Beschreibung

Die Erfindung betrifft eine Einrichtung zur überwachung leichtflüchtiger chlorierter und fluorierter Kohlenwasserstoffe in Abwässern mit einem Gasmeßgerät, an welchem an der Gaseintrittsseite über eine erste Luftleitung eine Entnahmevorrichtung mit einem Phasentauscher angeschlossen ist und mit einer zweiten Luftleitung, die in den Phasentauscher hineinragt, und durch die Luft zum Austreiben der im Abwasser nachzuweisenden Kohlenwasserstoffe gedrückt wird.

Bei einer bekannten Einrichtung zur Messung des Sauerstoffgehaltes in Abwasseraufbereitungs-Einrichtungen, wird die Abwasserprobe in einem in das Abwasser hineinragenden Rohr gesammelt, welches an beiden Enden offen ist und einen ständigen Zulauf von neuem Abwasser erhält (US-B-3 942 792). Eine Pumpe pumpt Luft durch einen Luftzerstäuber in das mit der Abwasserprobe gefüllte Rohr, und es setzen sich aus dem Abwasser eine Anzahl von aus dem Rohr nach oben in einen Behälter hinausgetriebene Luftblasen als Gas ab, das über eine Leitung einem Sauerstoffsensor zur Messung des Sauerstoffgehaltes zugeführt wird. Das gemessene Gas wird durch die Pumpe wieder dem Meßgas zugeführt.

Weiterhin ist ein Verfahren zur Wasserüberwachung bekannt, bei dem kontinuierlich ein Teilstrom des zu untersuchenden Wassers als Wasserprobe durch ein Extraktionsgefäß geleitet und einer kontinuierlich arbeitenden Meßstation zugeführt wird (DE-B 2 713 621), und zwar mit Hilfe eines perlenden Trägergases.

Schließlich ist eine von einem Hilfsgas durchströmte Phasenaustauschvorrichtung mit einem daran angeschlossenen Analysengerät bekannt, um einen kontinuierlichen Phasenaustausch zu erreichen und die Lösungskonzentration von in strömenden Flüssigkeiten gelösten Gasen messen zu können (DE-B-2 260 088).

Aufgabe der Erfindung ist es, eine Einrichtung zur Überwachung leichtflüchtiger chlorierter Kohlenwasserstoffe in Abwässern zu schaffen, bei der in vorteilhafter Weise als Überwachungsgerät ein Gasmeßgerät verwendet wird.

Diese Aufgabe wird durch die Einrichtung gemäß Anspruch 1 gelöst.

Die mit der Erfindung erzielten Vorteile bestehen insbesondere darin, daß ein zur Überwachung von Schadstoffen in der Umgebungsluft geeignetes Gasmeßgerät ebenfalls zur Überwachung von Schadstoffen in Abwässern eingesetzt werden kann, in dem mit einfachen Mitteln die Meßluft aufbereitet und an die Meßempfindlichkeit des Gasmeßgerätes angepaßt wird.

Ein Ausführungsbeispiel der Erfindung ist in der Zeichnung dargestellt und wird im folgenden näher beschrieben. Es zeigt:

Fig. 1     eine Darstellung der Überwachungseinrichtung,

Fig. 2     einen Ausschnitt der Entnahmevorrichtung, wobei ein Phasentauscher direkt im zu Überwachenden Abwasserkanal fest angeordnet ist,

Fig. 3     einen weiteren Ausschnitt der Entnahmeeinrichtung, wobei ein Phasentauscher als Schwimmer ausgebildet und direkt im zu überwachenden Abwasserkanal angeordnet ist.

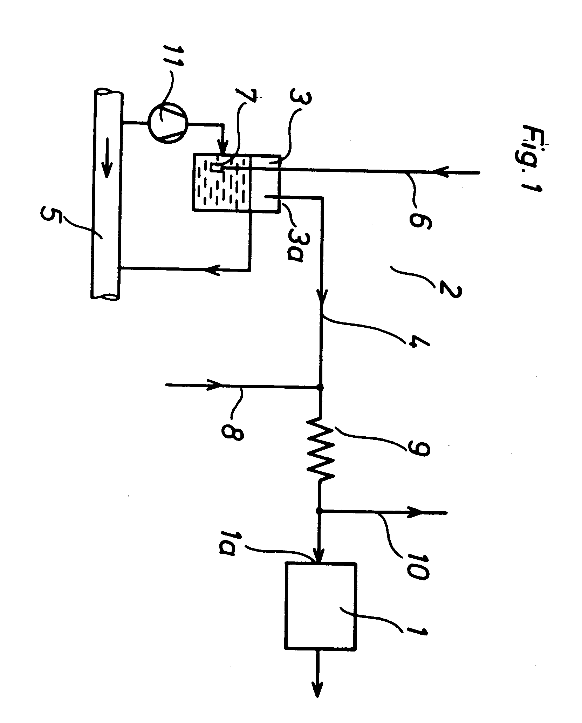

Wie aus der Fig. 1 ersichtlich ist, besteht die Überwachungsanlage im wesentlichen aus einem Gasmeßgerät 1 und aus einer dem Gasmeßgerät 1 vorgeschalteten Entnahmevorrichtung 2, wobei die Entnahmevorrichtung als Kernstück einen Phasentauscher 3 aufweist und über eine Luftleitung 4 an der Gaseintrittsseite 1a des Gasmeßgerätes angeschlossen ist.

In den vom Abwasser des Abwasserkanals 5 durchflossenen Phasentauscher 3, ragt eine Luftleitung 6 hinein, die an ihrem Ende von einer porösen Fritte 7 abgeschlossen ist. Durch die Luftleitung 6 wird sogenannte Spülluft zum Austreiben der im Abwasser nachzuweisenden leichtflüchtigen Kohlenwasserstoffe gedrückt, wobei die ausgetriebenen Kohlenwasserstoffenteile in einem Luftstrom durch die Luftleitung 4 als Meßluft dem Gasmeßgerät 1 zugeführt wird. Eine Pumpe 11 fördert kontinuierlich Wasserproben aus dem Abwasserkanal 5 in den Phasentauscher 3. Um die Meßluft den unterschiedlichsten Meßempfindlichkeiten der Gasmeßgeräte anpassen zu können, ist in der Luftleitung 4 zwischen dem Phasentauscher 3 und dem Gasmeßgerät 1 ein Rohrabzweig 8 und eine Mischstrecke 9 vorgesehen, in dem Luft zum Verdünnen der Meßluft eingeleitet wird.

Vor der Gaseintrittsseite 1a des Gasmeßgerätes 1 ist in der Leitung ein weiterer Rohrabzweig 10 angeschlossen, über den überschüssige Meßluft abgeführt werden kann.

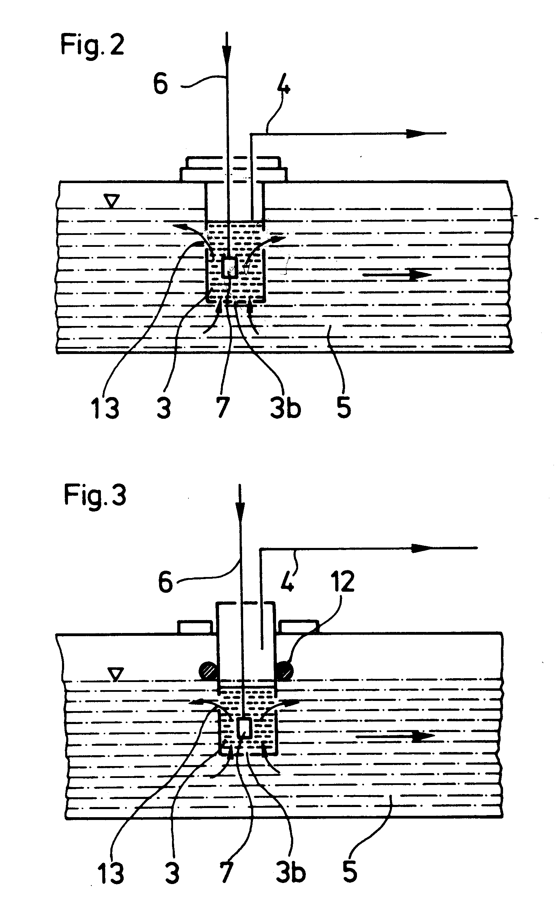

In der Fig. 2 ist eine vorteilhafte Ausführung des Phasentauschers 3 dargestellt, indem der Phasentauscher direkt an der zu überwachenden Entnahmestelle, d.h. in den Abwasserkanal 5 hineinragt und dort fest angeordnet ist. Am Umfang des Phasentausschers 3 sind Öffnungen 13 verteilt angeordnet. Durch teilweisen Austritt der Luft aus den Öffnungen 13, wird an der Unterseite 3b des Phasentauschers fortwährend frisches Meßmedium angesaugt und nach Phasentausch aus diesen öffnungen wieder herausgeführt. Insofern kann von einer sogenannten Selbstansaugung des Phasentauschers gesprochen werden.

In Fig. 3 ist in einer weiteren vorteilhaften Ausführung der Phasentauscher 3 in dem zu überwachenden Abwasserkanal 5 beweglich auf einem Schwimmer 12 angeordnet.

**Patentansprüche**

1. Einrichtung zur Überwachung leichtflüchtiger chlorierter und fluorierter Kohlenwasserstoffe in Abwässern mit einem Gasmeßgerät (1), an welchem an der Gaseintrittsseite (1a) über eine erste Luftleitung (4) eine Entnahmevorrichtung (2) mit einem Phasentauscher (3) angeschlossen ist und mit einer zweiten Luftleitung (6), die in den Phasentauscher hineinragt und durch die Luft zum Austreiben der im Abwasser nachzuweisenden Kohlenwasserstoffe gedrückt wird, dadurch gekennzeichnet, daß
   a) in der Luftleitung (4) zwischen dem Phasenaustauscher (3) und dem Gasmeßgerät (1) ein Rohrabzweig (8) und eine Mischstrecke (9) angeordnet sind, in die Luft zum Verdünnen der Meßluft eingeleitet wird, und
   b) in der Leitung (4) zwischen der Mischstrecke (9) und dem Gasmeßgerät (1) ein weiterer Rohrabzweig (10) angeordnet ist, mittels dessen die dem Gasmeßgerät zugeführte Meßluft mengenmäßig regulierbar ist.

2. Einrichtung nach Anspruch 1, dadurch gekennzeichnet, daß Öffnungen (13) am Umfang des Phasentauschers (3) verteilt angeordnet sind und die Unterseite (3b) des Phasentauschers offen ist.

3. Einrichtung nach Anspruch 1, dadurch gekennzeichnet, daß der Phasentauscher (3) auf einem Schwimmer angeordnet ist.

**Claims**

1. Apparatus for monitoring highly volatile chlorinated and fluorinated hydrocarbons in waste waters with a gas measuring device (1) to which on the gas inlet side (1a) an extraction device (2) with a phase exchanger (3) is connected by a first air pipe (4), and with a second air pipe (6) which extends into the phase exchanger and through which air is forced for driving out the hydrocarbons to be detected in the waste water, characterised in that
   a) in the air pipe (4) between the phase exchanger (3) and the gas measuring device (1) are arranged a pipe branch (8) and a mixing zone (9) into which air is introduced for thinning the measuring air, and
   b) in the pipe (4) between the mixing zone (9) and the gas measuring device (1) is arranged a further pipe branch (10) by means of which the measuring air supplied to the gas measuring device can be regulated with respect to quantity.

2. Apparatus according to claim 1, characterised in that openings (13) are distributed over the circumference of the phase exchanger (3) and the lower side (3b) of the phase exchanger is open.

3. Apparatus according to claim 1, characterised in that the phase exchanger (3) is arranged on a float.

**Revendications**

1. Appareillage pour surveiller la présence d'hydrocarbures chlorés et fluorés très volatils dans les eaux usées, comprenant un appareil mesureur de gaz (1), au côté d'entrée de gaz (1a) duquel est raccordé, par un premier conduit d'air (4), un dispositif de prélèvement (2) comportant un échangeur de phase (3), ainsi qu'un second conduit d'air (6) qui pénètre dans l'échangeur de phase et à travers duquel est refoulé de l'air pour chasser les hydrocarbures à détecter contenus dans l'eau usée, caractérisé en ce que
   a) un tuyau de dérivation (8) et une zone de mélange (9), servant à l'introduction d'air pour diluer l'air à mesurer, sont disposés dans le conduit d'air (4) entre l'échangeur de phase (3) et l'appareil mesureur de gaz (1) et
   b) un tuyau de dérivation supplémentaire (10) est disposé dans le conduit d'air (4) entre la zone de mélange (9) et l'appareil mesureur de gaz (1) pour réguler le débit de l'air à mesurer amené à l'appareil mesureur de gaz.

2. Appareillage selon la revendication 1, caractérisé en ce que des orifices (13) sont répartis à la périphérie de l'échangeur de phase (3) et le côté inférieur (3b) de l'échangeur de phase est ouvert.

3. Appareillage selon la revendication 1, caractérisé en ce que l'échangeur de phase (3) est placé sur un flotteur.

Fig. 1

Fig. 2

6     4

13    3    7    3b    5

Fig. 3

6     4    12

13    3    7    3b    5